(19) Europäisches Patentamt
European Patent Office
Office européen des brevets

(11) Veröffentlichungsnummer: **0 388 771 B1**

# (12) EUROPÄISCHE PATENTSCHRIFT

(45) Veröffentlichungstag der Patentschrift: **28.06.95**

(51) Int. Cl.⁶: **C07D 239/42**, C07D 239/52, C07D 239/47, C07D 239/56, C07D 251/44, C07D 251/46, C07D 251/42, A01N 47/36

(21) Anmeldenummer: **90104783.7**

(22) Anmeldetag: **14.03.90**

Die Akte enthält technische Angaben, die nach dem Eingang der Anmeldung eingereicht wurden und die nicht in dieser Patentschrift enthalten sind.

## (54) Phenoxysulfonylharnstoffe auf Basis von 3-substituierten Salicylsäurealkylestern, Verfahren zu ihrer Herstellung und ihre Verwendung als Herbizide und Pflanzenwachstumsregulatoren.

(30) Priorität: **18.03.89 DE 3909053**

(43) Veröffentlichungstag der Anmeldung:
**26.09.90 Patentblatt 90/39**

(45) Bekanntmachung des Hinweises auf die Patenterteilung:
**28.06.95 Patentblatt 95/26**

(84) Benannte Vertragsstaaten:
**CH DE ES FR GB IT LI**

(56) Entgegenhaltungen:
**EP-A- 0 004 163**
**EP-A- 0 303 114**
**DE-A- 3 151 450**
**EP-A- 0 098 569**
**DE-A- 3 105 453**
**US-A- 4 391 976**

(73) Patentinhaber: **Hoechst Schering AgrEvo GmbH**
**Gerichtstrasse 27**
**D-13342 Berlin (DE)**

(72) Erfinder: **Kehne, Heinz, Dr.**
**Berliner Strasse 10**
**D-6238 Hofheim am Taunus (DE)**
Erfinder: **Willms, Lothar, Dr.**
**Lindenstrasse 17**
**D-5416 Hillscheid (DE)**
Erfinder: **Bauer, Klaus, Dr.**
**Doorner Strasse 53d**
**D-6450 Hanau (DE)**
Erfinder: **Bieringer, Hermann, Dr.**
**Eichenweg 26**
**D-6239 Eppstein/Taunus (DE)**
Erfinder: **Bürstell, Helmut, Dr.**
**Am Hohlacker 65**
**D-6230 Frankfurt am Main 50 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Es ist bekannt, daß heterocyclisch substituierte Phenoxysulfonylharnstoffe herbizide und pflanzenwachstumsregulierende Eigenschaften besitzen (EP-A-4 163, DE-A-31 51 450, DE-A-37 25 939 (ZA-88/5725), Deutsche Patentanmeldung P-38 16 704.2 (EP-A-0342569, ZA-89/3643)).

So beschreibt EP-A-4 163 unter anderem 2-Methoxyphenoxy-, 2-Chlorphenoxy- und 2-Alkylphenoxy- sowie 2-Carboalkoxyphenoxysulfonylharnstoffe mit herbizider Wirkung.

Überraschenderweise wurde nun gefunden, daß die Kombination des Carboalkoxysubstituenten mit jeweils einem weiteren der genannten Reste zu einer erheblichen Verbesserung der herbiziden Eigenschaften führt.

Gegenstand der vorliegenden Erfindung sind daher Verbindungen der Formel (I) oder deren Salze

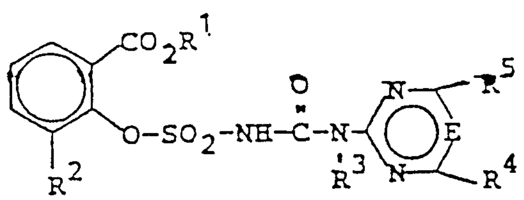

(I)

worin

- $R^1$ $(C_1\text{-}C_4)$Alkyl,
- $R^2$ Halogen, Methoxy, Ethyl oder Propyl,
- $R^3$ Wasserstoff oder Methyl,
- E CH oder N,
- $R^4$, $R^5$ unabhängig voneinander Halogen, $(C_1\text{-}C_4)$Alkyl, $(C_1\text{-}C_4)$Alkoxy oder $(C_1\text{-}C_4)$Alkylthio, wobei die vorgenannten alkylhaltigen Reste unsubstituiert sind oder ein- oder mehrfach durch Halogen oder ein- oder zweifach durch $(C_1\text{-}C_4)$Alkoxy oder $(C_1\text{-}C_4)$Alkylthio substituiert sind,

bedeuten.

Halogen bedeutet Fluor, Chlor, Brom und/oder Jod, vorzugsweise Fluor, Chlor und/oder Brom, insbesondere Fluor oder Chlor.

$(C_1\text{-}C_4)$Alkyl und der entsprechende Alkylrest in den alkylhaltigen Resten, wie Alkoxy oder Alkylthio, bedeutet Methyl, Ethyl, n-Propyl, i-Propyl, n-Butyl, i-Butyl, t-Butyl oder 2-Butyl.

Die Verbindungen der Formel I können Salze bilden, bei denen der Wasserstoff der $-SO_2$-NH-Gruppe durch ein für die Landwirtschaft geeignetes Kation ersetzt wird. Diese Salze sind im allgemeinen Metall-, insbesondere Alkali- oder Erdalkalimetallsalze, gegebenenfalls anorganische oder organische Ammoniumsalze.

Bevorzugte Verbindungen der Formel I oder deren Salze sind solche, bei denen $R^1$ Methyl oder Ethyl, E eine Gruppe der Formel CH, $R^4$ und $R^5$ unabhängig voneinander Chlor, Brom, $(C_1\text{-}C_4)$Alkyl oder $(C_1\text{-}C_4)$-Alkoxy, wobei die vorgenannten alkylhaltigen Reste ein- oder mehrfach durch Fluor oder Chlor substituiert sein können, bedeuten.

Besonders bevorzugte Verbindungen der Formel I oder deren Salze sind solche, worin $R^1$ Methyl oder Ethyl, E eine Gruppe der Formel CH, $R^4$ und $R^5$ unabhängig voneinander Chlor, $(C_1\text{-}C_2)$Alkyl oder $(C_1\text{-}C_2)$-Alkoxy, wobei die vorgenannten alkylhaltigen Reste ein- oder mehrfach durch Fluor oder Chlor substituiert sein können, bedeuten.

Weiterer Gegenstand der vorliegenden Erfindung sind Verfahren zur Herstellung der Verbindungen der allgemeinen Formel I oder deren Salze, dadurch gekennzeichnet, daß man

a) eine Verbindung der Formel (II)

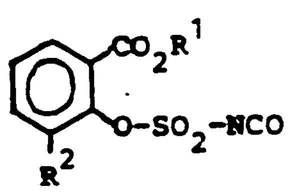

(II)

mit einer Verbindung der Formel (III)

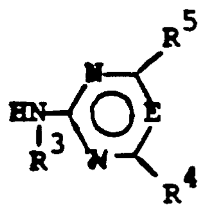

(III)

umsetzt, oder
b) eine Verbindung der Formel (IV)

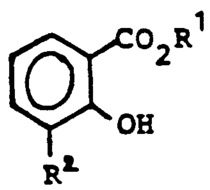

(IV)

mit einem Chlorsulfonylharnstoff der Formel (V)

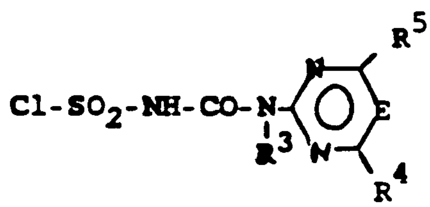

(V)

umsetzt, oder
c) eine Verbindung der Formel (VI)

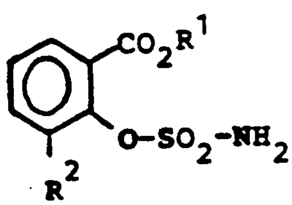

(VI)

mit einem Carbamat der Formel (VII)

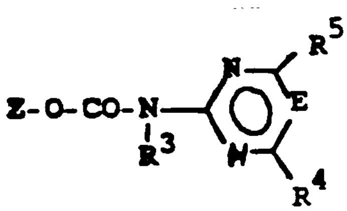

(VII)

worin Z Phenyl oder ($C_1$-$C_6$)Alkyl bedeutet, umsetzt, und die erhaltenen Verbindungen der Formel (I) gegebenenfalls in ihre Salze überführt.

Die Umsetzung der Verbindungen der Formeln (II) und (III) erfolgt vorzugsweise in inerten aprotischen Lösungsmitteln, wie z. B. Acetonitril, Dichlormethan, Toluol, Chlorbenzol, Tetrahydrofuran oder Dioxan bei Temperaturen zwischen 0 °C und der Siedetemperatur des Lösungsmittels.

Die Phenoxysulfonylisocyanate der Formel (II) lassen sich nach im Prinzip bekannten Verfahren aus den entsprechenden Salicylsäureestern der Formel (IV) und Chlorsulfonylisocyanat in einfacher Weise herstellen (vgl. G. Lohaus, Chem. Ber. 105, 2791 (1972)).

Die Ausgangsstoffe der Formel (III) sind bekannt oder lassen sich nach im Prinzip bekannten Verfahren herstellen, z. B. durch Cyclisierung entsprechender Guanidinderivate mit entsprechend substituierten 1,3-Diketonen, vergleiche z. B. "The Chemistry of Heterocyclic Compounds", Vol. XVI (1962) and Supplement I (1970), oder durch Derivatisierung von Cyanurchlorid, vgl. z. B. "The Chemistry of Heterocyclic Compounds", L. Rapaport: "s-Triazines and Derivatives" (1959).

Die Umsetzung der Verbindungen der Formel (IV) mit den Chlorsulfonylharnstoffen der Formel (V) führt man vorzugsweise in inerten Lösungsmitteln wie z. B. Dichlormethan, Tetrahydrofuran, Dioxan oder Dimethoxyethan bei Temperaturen zwischen -10 °C und 80 °C in Gegenwart einer Base als HCl-bindendes Agens durch. Als Basen können Alkali- oder Erdalkalicarbonate bzw. -bicarbonate wie z. B. $K_2CO_3$, $NaHCO_3$, $Na_2CO_3$ oder tertiäre Amine wie z. B. Pyridin oder Triethylamin eingesetzt werden.

Die Salicylsäureester der Formel (IV) sind literaturbekannt oder können nach literaturbekannten Verfahren hergestellt werden. Die Chlorsulfonylharnstoffe der Formel (V) sind aus den Aminen der Formel (III) und Chlorsulfonylisocyanat zugänglich (EP-A 141 199).

Die Umsetzung der Verbindungen der Formel (VI) mit den heterocyclischen Carbamaten der Fomrel (VII) wird vorzugsweise in Gegenwart von tertiären organischen Basen wie z. B. 1,8-Diazabicyclo[5.4.0]-undec-7-en (DBU) in inerten Lösungsmitteln wie Acetonitril oder Dioxan bei Temperaturen zwischen 20 °C und der Siedetemperatur des Lösungsmittels durchgeführt (analog EP-A 44 807).

Die hierzu erforderlichen Carbamate der Formel (VII) sind literaturbekannt oder werden nach bekannten Verfahren hergestellt (EP-A 70 804). Die Sulfamate der Formel VI werden nach bekannten Verfahren aus den zugrundeliegenden Salicylsäureestern hergestellt (vgl. z.B. Synthesis 1978, 357; Z. Chem. 15, 270 (1975); Chem. Ber. 105, 2791 (1972)).

Die Salze der Verbindungen der Formel (I) werden vorzugsweise in inerten Lösungsmitteln wie z.B. Wasser, Methanol oder Aceton bei Temperaturen von 0 - 100 °C hergestellt. Geeignete Basen zur Herstellung der erfindungsgemäßen Salze sind beispielsweise Alkalicarbonate, wie Kaliumcarbonat, Alkali- und Erdalkalihydroxide, Ammoniak oder Ethanolamin.

Die erfindungsgemäßen Verbindungen der Formel I weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöckchen oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z. B. Avena, Lolium, Alopecurus, Phalaris, Echinochloa, Digitaria, Setaria etc. sowie Cyperusarten aus der annuellen Gruppe und auf seiten der perennierenden Spezies Agropyron, Cynodon, Imperata sowie Sorghum etc. und auch ausdauernde Cyperusarten gut erfaßt.

Bei dikotylen Unkrautarten erstreckt sich das Wirkungsspektrum auf Galium, Viola, Veronica, Lamium, Stellaria, Amaranthus, Sinapis, Ipomoea, Matricaria, Abutilon, Sida etc. auf der annuellen Seite sowie Convolvulus, Cirsium, Rumex, Artemisia etc. bei den perennierenden Unkräutern.

Unter den spezifischen Kulturbedingungen im Reis vorkommenden Unkräuter wie z. B. Sagittaria, Alisma, Eleocharis, Scirpus, Cyperus etc. werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert, oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein, und die Unkrautpflanzen bleiben in dem zum Applikationszeitpunkt vorhandenen Wuchsstadium stehen oder sterben nach einer gewissen Zeit mehr oder weniger schnell ab, so daß auf diese Weise eine für die Kulturpflanzen schädliche Unkrautkonkurrenz sehr früh und nachhaltig durch den Einsatz der neuen erfindungsgemäßen Mittel beseitigt werden kann.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrüber, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüberhinaus weisen die erfindungsgemäßen Verbindungen wachstumsregulatorische Eigenschaften bei Kulturpflanzen auf. Sie greifen regulierend in den pflanzeneigenen Stoffwechsel ein und können damit

zur Ernteerleichterung wie z.B. durch Auslösen von Desikkation, Abszission und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), emulgierbare Konzentrate (EC), wäßrige Lösungen (SL); Emulsionen, versprühbare Lösungen, Dispersionen auf Öl- oder Wasserbasis (SC), Suspoemulsionen, Stäubemittel (DP), Beizmittel, Granulate (GR) wie Boden-bzw. Streugranulate (FG) oder wasserdispergierbare Granulate (WG), ULV-Formulierungen, Mikrokapseln oder Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986; van Falenkenberg, "Pesticides Formulations", Marcel Dekker N.Y., 2nd Ed. 1972-73; K. Martens, "Spray Drying Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v. Olphen, "Introduction to Clay Colloid Chemistry"; 2nd Ed., J. Wiley & Sons, N.Y.; Marschen, "Solvents Guide"; 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Spritzpulver sind in Wasser gleichmäßig dispergierbare Präparate, die neben dem Wirkstoff außer gegebenenfalls einem Verdünnungs- oder Inertstoff noch Netzmittel, z.B. polyoxethylierte Alkylphenole, polyoxethylierte Fettalkohole und Fettamine, Alkansulfonate oder Alkylarylsulfonate wie Alkylbenzolsulfonate und Dispergiermittel, z.B. ligninsulfonsaures Natrium, 2,2′-dinaphthylmethan-6,6′-disulfonsaures Natrium, dibutylnaphthalinsulfonsaures Natrium oder auch oleoylmethyltaurinsaures Natrium enthalten. Die Herstellung erfolgt in üblicher Weise, z.B. durch Mahlen und Vermischen der Komponenten.

Emulgierbare Konzentrate können z.B. durch Auflösen des Wirkstoffes in einem inerten organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt werden. Bei flüssigen Wirkstoffen kann der Lösungsmittelanteil auch ganz oder teilweise entfallen. Als Emulgatoren können beispielsweise verwendet werden: Alkylarylsulfonsaure Calciumsalze wie Ca-dodecylbenzolsulfonat oder nichtionische Emulgatoren wie Fettsäurepolyglykolester, Alkyl-arylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyglykolether, Sorbitanfettsäureester, Polyoxethylensorbitanfettsäureester oder Polyoxethylensorbitester.

Stäubemittel kann man durch Vermahlen des Wirkstoffes mit feinverteilten, festen Stoffen z.B. Talkum, natürlichen Tonen wie Kaolin, Bentonit, Pyrophillit oder Diatomeenerde erhalten.

Granulate können entweder durch Verdüsen des Wirkstoffes auf adsorptionsfähiges, granuliertes Inertmaterial hergestellt werden oder durch Aufbringen von Wirkstoffkonzentraten mittels Bindemitteln, z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise, gewünschtenfalls in Mischung mit Düngemitteln, granuliert werden.

Die agrochemischen Zubereitungen enthalten in der Regel 0,1 bis 99 Gewichtsprozent, insbesondere 2 bis 95 Gew.-%, Wirkstoff der Formel (I). Dabei können die Konzentrationen der Wirkstoff in Abhängigkeit vom Formulierungstyp verschieden sein.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-%, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe oder Gemische davon.

Gegenstand der Erfindung sind deshalb auch herbizide und pflanzenwachstumsregulierende Mittel, dadurch gekennzeichnet, daß sie eine Verbindung der Formel (I) oder deren Salze und übliche unter den Lagerbedingungen inerte Formulierungshilfsmittel enthalten.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,005 und 10,0 kg/ha oder mehr Aktivsubstanz, vorzugsweise liegt sie jedoch zwischen 0,01 und 5 kg/ha.

Auch Mischungen oder Mischformulierungen mit anderen Wirkstoffen, wie z.B. Insektiziden, Akariziden, Herbiziden, Düngemitteln, Wachstumsregulatoren oder Fungiziden sind gegebenenfalls möglich.

Die Erfindung wird durch nachstehende Beispiele näher erläutert.

**Formulierungsbeispiele**

A. Ein Stäubemittel wird erhalten, indem man 10 Gewichtsteile Wirkstoff mit 90 Gewichtsteilen Talkum oder einen vergleichbaren Snertstoff mischt und in einer Schlagmühle zerkleinert.

B. Ein in Wasser leicht dispergierbares, benetzbares Pulver wird erhalten, indem man 25 Gewichtsteile Wirkstoff, 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.

C. Ein in Wasser leicht dispergierbares Dispersionskonzentrat wird erhalten, indem man 20 Gewichtsteile Wirkstoff mit 6 Gewichtsteilen Alkylphenolpolyglykolether ((R)Triton X 207), 3 Gewichtsteilen Isotridecanolpolyglykolether (8 EO) und 71 Gewichtsteilen paraffinischem Mineralöl (Siedebereich z.B. ca. 255 bis über 377 °C) mischt und in einer Reibkugelmühle auf eine Feinheit von unter 5 Mikron vermahlt.

D. Ein emulgierbares Konzentrat wird erhalten aus 15 Gewichtsteilen Wirkstoff, 75 Gewichtsteilen Cyclohexanon als Lösungsmittel und 10 Gewichtsteilen oxethyliertes Nonylphenol (10 EO) als Emulgator.

E. Ein in Wasser dispergierbares Granulat wird erhalten, indem man
75 Gewichtsteile einer Verbindung der Formel (I),
10 Gewichtsteile ligninsulfonsaures Calcium,
5 Gewichtsteile Natriumlaurylsulfat,
3 Gewichtsteile Polyvinylalkohol und
7 Gewichtsteile Kaolin
mischt, auf einer Stiftmühle mahlt und das Pulver in einem Wirbelbett durch Aufsprühen von Wasser als Granulierflüssigkeit granuliert.

F. Ein in Wasser dispergierbares Granulat wird auch erhalten, indem man
25 Gewichtsteile einer Verbindung der Formel (I),
5 Gewichtsteile 2,2′-dinaphthylmethyl-6,6′-disulfonsaures Natrium,
2 Gewichtsteile oleolymethyltaurinsaures Natrium,
1 Gewichtsteile Polyvinylalkohol,
17 Gewichtsteile Calciumcarbonat und
50 Gewichtsteile Wasser
auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

G. Ein Extruder-Granulat erhält man, indem man
20 Gewichtsteile Wirkstoff,
3 Gewichtsteile ligninsulfonsaures Natrium,
1 Gewichtsteile Carboxymethylcellulose und
76 Gewichtsteile Kaolin
vermischt, vermahlt und mit Wasser anfeuchtet. Dieses Gemisch wird extrudiert und anschließend in Luftstom getrocknet.

## Chemische Beispiele

**Beispiel 1** (Vorprodukt)

**2- Isocyanatosulfonyloxy- 3-methoxy-benzoesäuremethylester**

Zu einer Lösung von 3,6 g (0,02 mol) 3-Methoxy-salicylsäuremethylester in 20 ml Xylol läßt man bei 25 °C 3,4 g (0,024 mol) Chlorsulfonylisocyanat tropfen. Nach Beendigung des Zutropfens steigert man die Temperatur langsam auf 140 °C und erhitzt 2,5 h unter Rückfluß. Man kühlt ab und entfernt das Lösungsmittel sowie überschüssiges Chlorsulfonylisocyanat am Rotationsverdampfer. Das zurückbleibende gelbe Öl (5,4 g = 100 % d.Th.) wird ohne weitere Reinigung eingesetzt.

**Beispiel 2**

**2-[3-(4,6-Dimethoxypyrimidin-2-yl)-ureidosulfonyloxy]-3-methoxy-benzoesäuremethylester (Formel I mit $R^1$ = $CH_3$, $R^2$ = $OCH_3$, $R^3$ = H, $R^4$ = $OCH_3$, $R^5$ = $OCH_3$, E = CH)**

Zu 3,1 g (0,02 mol) 2-Amino-4,6-dimethoxypyrimidin in 50 ml Dichlormethan läßt man bei 0 °C eine Lösung von 5,4 g (0,02 mol) des Produktes aus Beispiel 1 in 10 ml Dichlormethan tropfen. Man rührt 24 h bei 25 °C nach, verdünnt mit 50 ml Dichlormethan und wäscht die organische Phase 2 x mit je 50 ml 2N Salzsäure und 1 x mit 50 ml Wasser. Nach Trocknen mit Natriumsulfat und Entfernen des Lösungsmittels am Rotationsverdampfer hinterbleibt ein öliges Produkt, das beim Verreiben mit Diethylether kristallisiert. Man erhält 6,8 g (77 d.Th.) 2-[3-(4,6-Dimethoxypyrimidin-2-yl)-ureidosulfonyloxy]-3-methoxy-benzoesäuremethylester vom Schmp. 169-170 °C.

**Beispiel 3**

**2-[3-(4-Chlor-6-methylpyrimidin-2-yl)-ureidosulfonyloxy]-3-methoxybenzoesäuremethylester (Formel I mit $R^1$ = $CH_3$, $R^2$ = $OCH_3$, $R^3$ = H, $R^4$ = Cl, $R^5$ = $CH_3$, E = CH)**

Zu 2,9 g (0,02 mol) 2-Amino-4-chlor-6-methylpyrimidin in 50 ml Dichlormethan läßt man bei 0 °C eine ,Lösung von 5,4 g (0,02 mol) des Produktes aus Beispiel 1 in 10 ml Dichlormethan tropfen. Man rührt 24 h bei 25 °C nach, verdünnt mit 50 ml Dichlormethan und wäscht die organische Phase zweimal mit je 50 ml 2N Salzsäure und einmal mit Wasser. Nach Trocknen mit Natriumsulfat und Entfernen des Lösungsmittels am Rotationsverdampfer hinterbleibt ein öliges Produkt, das beim Verreiben mit Diisopropylether kristallisiert. Man erhält 7,8 g (91 % d.Th.) 2-[3-(4-Chlor-6-methylpyrimidin-2-yl)-ureidosulfonyloxy]-3-methoxy-benzoesäuremethylester vom Schmp. 140-143 °C.

Die nachfolgenden in Tabelle 1 aufgeführten Verbindungen können wie in den Beispielen 1 - 3 beschrieben hergestellt werden.

Tabelle 1

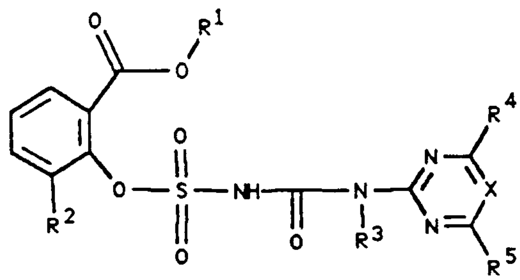

| Bsp.Nr. | $R^1$ | $R^2$ | $R^3$ | $R^4$ | $R^5$ | X | Fp. [°C] |
|---|---|---|---|---|---|---|---|
| 4 | $CH_3$ | $OCH_3$ | H | $CH_3$ | $CH_3$ | CH | 144-145 |
| 5 | " | " | H | $OCH_3$ | $CH_3$ | CH | 147-149 |
| 6 | " | " | H | $CH_3$ | $CH_3$ | N | |
| 7 | " | " | H | $OCH_3$ | $CH_3$ | N | 160-161 |
| 8 | " | " | H | $OCH_3$ | $OCH_3$ | N | |
| 9 | " | " | H | $OCH_3$ | Cl | CH | 130-133 |
| 10 | " | " | H | $OCF_2H$ | $CH_3$ | CH | |
| 11 | " | " | H | $OCF_2H$ | $OCF_2H$ | CH | |
| 12 | " | " | H | $OCH_3$ | Br | CH | |
| 13 | " | " | H | $OCH_3$ | $OC_2H_5$ | CH | |
| 14 | " | " | H | $OCH_3$ | $SCH_3$ | CH | |
| 15 | " | " | H | $OCH_3$ | $OC_2H_5$ | N | |
| 16 | " | " | H | $OCH_3$ | $OC_3H_7$ | CH | |
| 17 | " | " | H | $OCH_3$ | Cl | N | |
| 18 | " | " | H | Cl | $OC_2H_5$ | CH | |
| 19 | " | " | H | $OC_2H_5$ | $OC_2H_5$ | CH | |
| 20 | " | " | H | $C_2H_5$ | $OCH_3$ | CH | |
| 21 | " | " | H | $CF_3$ | $OCH_3$ | CH | |
| 22 | " | " | H | $OCH_2CF_3$ | $CH_3$ | CH | |
| 23 | " | " | H | $OCH_2CF_3$ | $OCH_3$ | CH | |
| 24 | " | " | H | $OCH_2CF_3$ | $OCH_2CF_3$ | CH | |
| 25 | " | " | H | $OCH_2CF_3$ | $OCH_3$ | N | 143-144 |
| 27 | " | " | H | $OCH_3$ | $CH(OCH_3)_2$ | CH | |
| 28 | $C_2H_5$ | " | H | $OCH_3$ | $OCH_3$ | CH | |
| 29 | " | " | H | $OCH_3$ | $CH_3$ | CH | |
| 30 | " | " | H | $CH_3$ | $CH_3$ | CH | |

| | | | | | | | |
|---|---|---|---|---|---|---|---|
| 31 | " | " | H | $OCH_3$ | Cl | CH | |
| 32 | " | " | H | $OCH_3$ | $OCH_3$ | N | |
| 33 | " | " | H | $OCH_3$ | $CH_3$ | N | |
| 34 | $C_3H_7$ | " | H | $OCH_3$ | $OCH_3$ | CH | |
| 35 | " | " | H | $OCH_3$ | $CH_3$ | CH | |
| 36 | " | " | H | $OCH_3$ | $CH_3$ | N | |
| 37 | $C_4H_9$ | " | H | $OCH_3$ | $OCH_3$ | CH | 129 |
| 38 | " | " | H | $OCH_3$ | $CH_3$ | CH | 118-119 |
| 39 | " | " | H | $OCH_3$ | $CH_3$ | N | |
| 40 | $CH_3$ | " | $CH_3$ | $OCH_3$ | $OCH_3$ | CH | 144-145 |
| 41 | " | " | $CH_3$ | $OCH_3$ | $CH_3$ | CH | |
| 42 | " | " | $CH_3$ | $OCH_3$ | $CH_3$ | N | 100-103 |
| 43 | " | " | $CH_3$ | $OCH_3$ | Cl | CH | |
| 44 | " | " | $CH_3$ | $OCF_2H$ | $OCF_2H$ | CH | |
| 45 | " | " | $CH_3$ | $CH_3$ | $CH_3$ | CH | |
| 46 | $C_2H_5$ | " | $CH_3$ | $OCH_3$ | $OCH_3$ | CH | |
| 47 | " | " | $CH_3$ | $OCH_3$ | $CH_3$ | CH | |
| 48 | $CH_3$ | Cl | H | $CH_3$ | $CH_3$ | CH | |
| 49 | " | " | H | $OCH_3$ | $CH_3$ | CH | 92- 95 (Zers.) |
| 50 | " | " | H | $CH_3$ | $CH_3$ | N | |
| 51 | " | " | H | $OCH_3$ | $CH_3$ | N | |
| 52 | " | " | H | $OCH_3$ | $OCH_3$ | N | |
| 53 | " | " | H | $OCH_3$ | Cl | CH | |
| 54 | " | " | H | $OCF_2H$ | $CH_3$ | CH | |
| 55 | " | " | H | $OCF_2H$ | $OCF_2H$ | CH | |
| 56 | " | " | H | $OCH_3$ | Br | CH | |
| 57 | " | " | H | $OCH_3$ | $OC_2H_5$ | CH | |
| 58 | " | " | H | $OCH_3$ | $SCH_3$ | CH | |
| 59 | " | " | H | $OCH_3$ | $OC_2H_5$ | N | |
| 60 | " | " | H | $OCH_3$ | $OC_3H_7$ | CH | |
| 61 | " | " | H | $OCH_3$ | Cl | N | |
| 62 | " | " | H | Cl | $OC_2H_5$ | CH | |
| 63 | " | " | H | $OC_2H_5$ | $OC_2H_5$ | CH | |
| 64 | " | " | H | $C_2H_5$ | $OCH_3$ | CH | |
| 65 | " | " | H | $CF_3$ | $OCH_3$ | CH | |
| 66 | " | " | H | $OCH_2CF_3$ | $CH_3$ | CH | |
| 67 | " | " | H | $OCH_2CF_3$ | $OCH_3$ | CH | |
| 68 | " | " | H | $OCH_2CF_3$ | $OCH_2CF_3$ | CH | |
| 69 | " | " | H | $OCH_2CF_3$ | $OCH_3$ | N | |
| 70 | " | " | H | $OCH_3$ | $CH(OCH_3)_2$ | CH | |

| | | | | | | | |
|---|---|---|---|---|---|---|---|
| 71 | $C_2H_5$ | " | H | $OCH_3$ | $OCH_3$ | CH | |
| 72 | " | " | H | $OCH_3$ | $CH_3$ | CH | |
| 73 | " | " | H | $CH_3$ | $CH_3$ | CH | |
| 74 | " | " | H | $OCH_3$ | Cl | CH | |
| 75 | " | " | H | $OCH_3$ | $OCH_3$ | N | |
| 76 | " | " | H | $OCH_3$ | $CH_3$ | N | |
| 77 | $C_3H_7$ | " | H | $OCH_3$ | $OCH_3$ | CH | |
| 78 | " | " | H | $OCH_3$ | $CH_3$ | CH | |
| 79 | " | " | H | $OCH_3$ | $CH_3$ | N | |
| 80 | $C_4H_9$ | " | H | $OCH_3$ | $OCH_3$ | CH | |
| 81 | " | " | H | $OCH_3$ | $CH_3$ | CH | |
| 82 | " | " | H | $OCH_3$ | $CH_3$ | N | |
| 83 | $CH_3$ | " | $CH_3$ | $OCH_3$ | $OCH_3$ | CH | |
| 84 | " | " | $CH_3$ | $OCH_3$ | $CH_3$ | CH | |
| 85 | " | " | $CH_3$ | $OCH_3$ | $CH_3$ | N | |
| 86 | " | " | $CH_3$ | $OCH_3$ | Cl | CH | |
| 87 | " | " | $CH_3$ | $OCF_2H$ | $OCF_2H$ | CH | |
| 88 | " | " | $CH_3$ | $CH_3$ | $CH_3$ | CH | |
| 89 | $C_2H_5$ | " | $CH_3$ | $OCH_3$ | $OCH_3$ | CH | |
| 90 | " | " | $CH_3$ | $OCH_3$ | $CH_3$ | CH | |
| 91 | $CH_3$ | " | H | $OCH_3$ | $OCH_3$ | CH | 134-135 |
| 92 | " | " | H | $CH_3$ | Cl | CH | |
| 93 | $CH_3$ | $C_2H_5$ | H | $CH_3$ | $CH_3$ | CH | |
| 94 | " | " | H | $OCH_3$ | $CH_3$ | CH | 140-141 |
| 95 | " | " | H | $CH_3$ | $CH_3$ | N | |
| 96 | " | " | H | $OCH_3$ | $CH_3$ | N | |
| 97 | " | " | H | $OCH_3$ | $OCH_3$ | N | |
| 98 | " | " | H | $OCH_3$ | Cl | CH | |
| 99 | " | " | H | $OCF_2H$ | $CH_3$ | CH | |
| 100 | " | " | H | $OCF_2H$ | $OCF_2H$ | CH | |
| 101 | " | " | H | $OCH_3$ | Br | CH | |
| 102 | " | " | H | $OCH_3$ | $OC_2H_5$ | CH | |
| 103 | " | " | H | $OCH_3$ | $SCH_3$ | CH | |
| 104 | " | " | H | $OCH_3$ | $OC_2H_5$ | N | |
| 105 | " | " | H | $OCH_3$ | $OC_3H_7$ | CH | |
| 106 | " | " | H | $OCH_3$ | Cl | N | |
| 107 | " | " | H | Cl | $OC_2H_5$ | CH | |
| 108 | " | " | H | $OC_2H_5$ | $OC_2H_5$ | CH | |
| 109 | " | " | H | $C_2H_5$ | $OCH_3$ | CH | |
| 110 | " | " | H | $CF_3$ | $OCH_3$ | CH | |

| | | | | | | | |
|---|---|---|---|---|---|---|---|
| 111 | " | " | H | $OCH_2CF_3$ | $CH_3$ | CH | |
| 112 | " | " | H | $OCH_2CF_3$ | $OCH_3$ | CH | |
| 113 | " | " | H | $OCH_2CF_3$ | $OCH_2CF_3$ | CH | |
| 114 | " | " | H | $OCH_2CF_3$ | $OCH_3$ | N | |
| 115 | " | " | H | $OCH_3$ | $CH(OCH_3)_2$ | CH | |
| 116 | $C_2H_5$ | " | H | $OCH_3$ | $OCH_3$ | CH | |
| 117 | " | " | H | $OCH_3$ | $CH_3$ | CH | |
| 118 | " | " | H | $CH_3$ | $CH_3$ | CH | |
| 119 | " | " | H | $OCH_3$ | Cl | CH | |
| 120 | " | " | H | $OCH_3$ | $OCH_3$ | N | |
| 121 | " | " | H | $OCH_3$ | $CH_3$ | N | |
| 122 | $C_3H_7$ | " | H | $OCH_3$ | $OCH_3$ | CH | |
| 123 | " | " | H | $OCH_3$ | $CH_3$ | CH | |
| 124 | " | " | H | $OCH_3$ | $CH_3$ | N | |
| 125 | $C_4H_9$ | " | H | $OCH_3$ | $OCH_3$ | CH | |
| 126 | " | " | H | $OCH_3$ | $CH_3$ | CH | |
| 127 | " | " | H | $OCH_3$ | $CH_3$ | N | |
| 128 | $CH_3$ | " | $CH_3$ | $OCH_3$ | $OCH_3$ | CH | |
| 129 | " | " | $CH_3$ | $OCH_3$ | $CH_3$ | CH | |
| 130 | " | " | $CH_3$ | $OCH_3$ | $CH_3$ | N | |
| 131 | " | " | $CH_3$ | $OCH_3$ | Cl | CH | |
| 132 | " | " | $CH_3$ | $OCF_2H$ | $OCF_2H$ | CH | |
| 133 | " | " | $CH_3$ | $CH_3$ | $CH_3$ | CH | |
| 134 | $C_2H_5$ | " | $CH_3$ | $OCH_3$ | $OCH_3$ | CH | |
| 135 | " | " | $CH_3$ | $OCH_3$ | $CH_3$ | CH | |
| 136 | $CH_3$ | " | H | $OCH_3$ | $OCH_3$ | CH | 137-138 |
| 137 | " | " | H | $CH_3$ | Cl | CH | |
| 138 | $CH_3$ | $C_3H_7$ | H | $CH_3$ | $CH_3$ | CH | |
| 139 | " | " | H | $OCH_3$ | $CH_3$ | CH | |
| 140 | " | " | H | $CH_3$ | $CH_3$ | N | |
| 141 | " | " | H | $OCH_3$ | $CH_3$ | N | |
| 142 | " | " | H | $OCH_3$ | $OCH_3$ | N | |
| 143 | " | " | H | $OCH_3$ | Cl | CH | |
| 144 | " | " | H | $OCF_2H$ | $CH_3$ | CH | |
| 145 | " | " | H | $OCF_2H$ | $OCF_2H$ | CH | |
| 146 | " | " | H | $OCH_3$ | Br | CH | |
| 147 | " | " | H | $OCH_3$ | $OC_2H_5$ | CH | |
| 148 | " | " | H | $OCH_3$ | $SCH_3$ | CH | |
| 149 | " | " | H | $OCH_3$ | $OC_2H_5$ | N | |
| 150 | " | " | H | $OCH_3$ | $OC_3H_7$ | CH | |

| | | | | | | |
|---|---|---|---|---|---|---|
| 151 | " | " | H | $OCH_3$ | Cl | N |
| 152 | " | " | H | Cl | $OC_2H_5$ | CH |
| 153 | " | " | H | $OC_2H_5$ | $OC_2H_5$ | CH |
| 154 | " | " | H | $C_2H_5$ | $OCH_3$ | CH |
| 155 | " | " | H | $CF_3$ | $OCH_3$ | CH |
| 156 | " | " | H | $OCH_2CF_3$ | $CH_3$ | CH |
| 157 | " | " | H | $OCH_2CF_3$ | $OCH_3$ | CH |
| 158 | " | " | H | $OCH_2CF_3$ | $OCH_2CF_3$ | CH |
| 159 | " | " | H | $OCH_2CF_3$ | $OCH_3$ | N |
| 160 | " | " | H | $OCH_3$ | $CH(OCH_3)_2$ | CH |
| 161 | $C_2H_5$ | " | H | $OCH_3$ | $OCH_3$ | CH |
| 162 | " | " | H | $OCH_3$ | $CH_3$ | CH |
| 163 | " | " | H | $CH_3$ | $CH_3$ | CH |
| 164 | " | " | H | $OCH_3$ | Cl | CH |
| 165 | " | " | H | $OCH_3$ | $OCH_3$ | N |
| 166 | " | " | H | $OCH_3$ | $CH_3$ | N |
| 167 | $C_3H_7$ | " | H | $OCH_3$ | $OCH_3$ | CH |
| 168 | " | " | H | $OCH_3$ | $CH_3$ | CH |
| 169 | " | " | H | $OCH_3$ | $CH_3$ | N |
| 170 | $C_4H_9$ | " | H | $OCH_3$ | $OCH_3$ | CH |
| 171 | " | " | H | $OCH_3$ | $CH_3$ | CH |
| 172 | " | " | H | $OCH_3$ | $CH_3$ | N |
| 173 | $CH_3$ | " | $CH_3$ | $OCH_3$ | $OCH_3$ | CH |
| 174 | " | " | $CH_3$ | $OCH_3$ | $CH_3$ | CH |
| 175 | " | " | $CH_3$ | $OCH_3$ | $CH_3$ | N |
| 176 | " | " | $CH_3$ | $OCH_3$ | Cl | CH |
| 177 | " | " | $CH_3$ | $OCF_2H$ | $OCF_2H$ | CH |
| 178 | " | " | $CH_3$ | $CH_3$ | $CH_3$ | CH |
| 179 | $C_2H_5$ | " | $CH_3$ | $OCH_3$ | $OCH_3$ | CH |
| 180 | " | " | $CH_3$ | $OCH_3$ | $CH_3$ | CH |
| 181 | $CH_3$ | " | H | $OCH_3$ | $OCH_3$ | CH |
| 182 | " | " | H | $CH_3$ | Cl | CH |

**Biologische Beispiele**

Die Schädigung der Unkrautpflanzen bzw. die Kulturpflanzenverträglichkeit wurde gemäß einem Schlüssel bonitiert, in dem die Wirksamkeit durch Wertzahlen von 0 bis 5 ausgedrückt ist. Dabei bedeutet:

0 = ohne Wirkung
1 = 0 bis 20 % Wirkung bzw. Schaden
2 = 20 bis 40 % Wirkung bzw. Schaden

3 = 40 bis 60 % Wirkung bzw. Schaden
4 = 60 bis 80 % Wirkung bzw. Schaden
5 = 80 bis 100 % Wirkung bzw. Schaden

**1. Unkrautwirkung im Vorauflauf**

Samen bzw. Rhizomstücke von mono- und dikotylen Unkrautpflanzen wurden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen wurden dann als wäßrige Suspensionen bzw. Emulsionen mit einer Wasseraufwandmenge von umgerechnet 600 bis 800 l/ha in unterschiedlichen Dosierungen auf die Oberfläche der Abdeckerde appliziert.

Nach der Behandlung wurden Töpfe im Gewächshaus aufgestellt und unter guten Wachstumsbedingungen für die Unkräuter gehalten. Die optische Bonitur der Pflanzen- bzw. der Auflaufschäden erfolgte nach dem Auflaufen der Versuchspflanzen nach einer Versuchszeit von 3 bis 4 Wochen im Vergleich zu unbehandelten Kontrollen. Wie die Boniturwerte in Tabelle 2 zeigen, weisen die erfindungsgemäßen Verbindungen eine gute herbizide Vorauflaufwirksamkeit gegen ein breites Spektrum von Ungräsern und Unkräutern auf.

**2. Unkrautwirkung im Nachauflauf**

Samen bzw. Rhizomstücke von mono- und dikotylen Unkräutern wurden in Plastiktöpfen in sandigem Lehmboden ausgelegt, mit Erde abgedeckt und im Gewächshaus unter guten Wachstumbedingungen angezogen. Drei Wochen nach der Aussaat wurden die Versuchspflanzen im Dreiblattstadium behandelt.

Die als Spritzpulver bzw. als Emulsionskonzentrate formulierten erfindungsgemäßen Verbindungen wurden in verschiedenen Dosierungen mit einer Wasseraufwandmenge von umgerechnet 600 bis 800 l/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert.

Die erfindungsgemäßen Mittel weisen auch im Nachauflauf eine gute herbizide Wirksamkeit gegen ein breites Spektrum wirtschaftlich wichtiger Ungräser und Unkräuter auf (Tabelle 3).

**3. Kulturpflanzenverträglichkeit**

In weiteren Versuchen im Gewächshaus wurden Samen einer größeren Anzahl von Kulturpflanzen und Unkräutern in sandigem Lehmboden ausgelegt und mit Erde abgedeckt.

Ein Teil der Töpfe wurde sofort wie unter 1. beschrieben behandelt, die übrigen im Gewächshaus aufgestellt, bis die Pflanzen zwei bis drei echte Blätter entwickelt hatten und dann mit den erfindungsgemäßen Substanzen in unterschiedlichen Dosierungen, wie unter 2. beschrieben besprüht.

Vier bis fünf Wochen nach der Applikation und Standzeit im Gewächshaus wurde mittels optischer Bonitur festgestellt, daß die erfindungsgemäßen Verbindungen zweikeimblättrige Kulturen wie z.B. Soja, Baumwolle, Raps, Zuckerrüben und Kartoffel im Vor- und Nachauflaufverfahren selbst bei hohen Wirkstoffdosierungen ungeschädigt ließen. Einige Substanzen schonten darüberhinaus auch Gramineen-Kulturen wie z.B. Gerste, Weizen, Roggen, Sorghum-Hirsen, Mais oder Reis. Die Verbindungen der Formel I weisen somit eine hohe Selektivität bei Anwendung zur Bekämpfung von unerwünschtem Pflanzenwuchs in landwirtschaftlichen Kulturen auf.

Tabelle 2

| Vorauflaufwirkung | | | | | |
|---|---|---|---|---|---|
| Bsp.- Nr. | Dosis kg a.i./ha | herbizide Wirkung | | | |
| | | SIAL | CRSE | LOMU | ECCR |
| 2 | 0,3 | 5 | 5 | 2 | 5 |
| 3 | 0,3 | 5 | 5 | 5 | 5 |
| 4 | 0,3 | 5 | 5 | 5 | 5 |
| 5 | 0,3 | 5 | 5 | 5 | 5 |
| 7 | 0,3 | 5 | 5 | 5 | 5 |
| 9 | 0,3 | 5 | 5 | 2 | 4 |
| 42 | 0,3 | 5 | 5 | 5 | 5 |
| 136 | 0,3 | 5 | 5 | 3 | 5 |
| 94 | 0,3 | 5 | 5 | 5 | 5 |
| 91 | 0,3 | 5 | 5 | 2 | 5 |
| 49 | 0,3 | 5 | 5 | 5 | 5 |
| 37 | 0,3 | 5 | 5 | 3 | - |
| 38 | 0,3 | 5 | 5 | 4 | - |
| 40 | 0,3 | 5 | 5 | 2 | 4 |

Tabelle 3

| Nachauflaufwirkung | | | | | |
|---|---|---|---|---|---|
| Bsp.- Nr. | Dosis kg a.i./ha | herbizide Wirkung | | | |
| | | SIAL | CRSE | LOMU | ECCR |
| 2 | 0,3 | 5 | 5 | 3 | 5 |
| 3 | 0,3 | 5 | 5 | 5 | 2 |
| 4 | 0,3 | 5 | 5 | 5 | 4 |
| 5 | 0,3 | 5 | 5 | 5 | 5 |
| 7 | 0,3 | 5 | 5 | 3 | 1 |
| 9 | 0,3 | 5 | 5 | 3 | 2 |
| 25 | 0,3 | 5 | 2 | 2 | 1 |
| 42 | 0,3 | 5 | 5 | 3 | 4 |
| 136 | 0,3 | 5 | 5 | 3 | 4 |
| 94 | 0,3 | 5 | 5 | 5 | 5 |
| 91 | 0,3 | 5 | 5 | 3 | 5 |
| 49 | 0,3 | 5 | 5 | 4 | 5 |
| 37 | 0,3 | 5 | 5 | 3 | 3 |
| 38 | 0,3 | 5 | 5 | 2 | 3 |
| 40 | 0,3 | 5 | 5 | 1 | 2 |

**Abkürzungen:**

SIAL = Sinapis alba
CRSE = Chrysanthemum segetum
LOMO = Lolium multiflorum
ECCR = Echinochloa crus-galli
a.i. = Aktivsubstanz

**Patentansprüche**

**Patentansprüche für folgende Vertragsstaaten : CH, DE, FR, GB, IT, LI**

1. Verbindungen der Formel (I) oder deren Salze

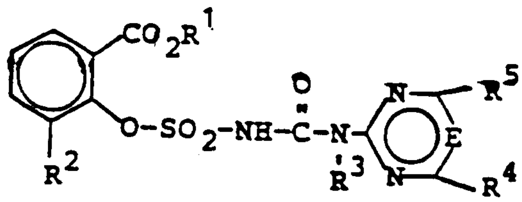

(I)

worin

| | |
|---|---|
| $R^1$ | $(C_1\text{-}C_4)$Alkyl, |
| $R^2$ | Halogen, Methoxy, Ethyl oder Propyl, |
| $R^3$ | Wasserstoff oder Methyl, |
| E | CH oder N und |
| $R^4$, $R^5$ | unabhängig voneinander Halogen, $(C_1\text{-}C_4)$Alkyl, $(C_1\text{-}C_4)$Alkoxy oder $(C_1\text{-}C_4)$Alkylthio, wobei die vorgenannten alkylhaltigen Reste unsubstituiert sind oder ein- oder mehrfach durch Halogen oder ein- oder zweifach durch $(C_1\text{-}C_4)$Alkoxy oder $(C_1\text{-}C_4)$Alkylthio substituiert sind, |

bedeuten.

2. Verbindungen nach Anspruch 1, dadurch gekennzeichnet, daß

| | |
|---|---|
| $R^1$ | Methyl oder Ethyl, |
| $R^2$ | Fluor, Chlor, Methoxy, Ethyl, n-Propyl oder Isopropyl, |
| $R^3$ | Wasserstoff oder Methyl, |
| E | CH und |
| $R^4$ und $R^5$ | unabhängig voneinander Chlor, Brom, $(C_1\text{-}C_4)$Alkyl oder $(C_1\text{-}C_4)$Alkoxy, wobei die alkylhaltigen Reste unsubstituiert sind oder ein- oder mehrfach durch Fluor oder Chlor substituiert sind, |

bedeuten.

3. Verbindungen nach Anspruch 1 oder 2, dadurch gekennzeichnet, daß $R^4$ und $R^5$ unabhängig voneinander Chlor, $(C_1\text{-}C_2)$Alkyl oder $(C_1\text{-}C_2)$Alkoxy bedeuten, wobei die vorgenannten alkylhaltigen Reste unsubstituiert sind oder ein- oder mehrfach durch Fluor oder Chlor substituiert sind.

4. Verfahren zur Herstellung der Verbindungen der allgemeinen Formel (I) nach einem oder mehreren der Ansprüche 1 bis 3 oder deren Salze, dadurch gekennzeichnet, daß man

a) eine Verbindung der Formel (II)

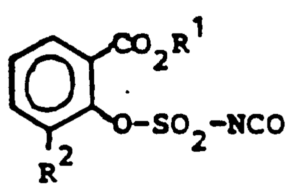

(II)

mit einer Verbindung der Formel (III)

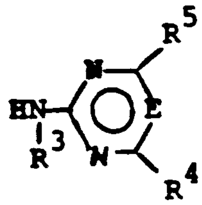

(III)

umsetzt, oder

b) eine Verbindung der Formel (IV)

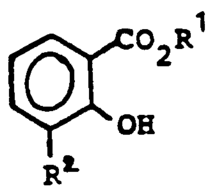

(IV)

mit einem Chlorsulfonylharnstoff der Formel (V)

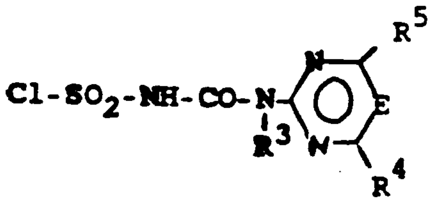

(V)

umsetzt, oder

c) eine Verbindung der Formel (VI)

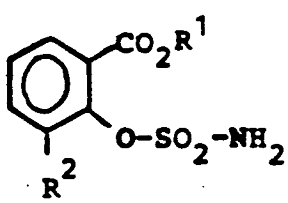

(VI)

mit einem Carbamat der Formel (VII)

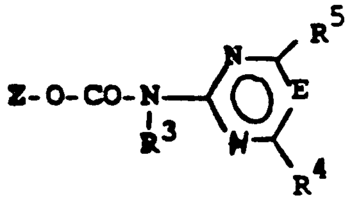

(VII),

worin Z Phenyl oder $(C_1\text{-}C_6)$Alkyl bedeutet, umsetzt, und die erhaltenen Verbindungen der Formel (I) gegebenenfalls in ihre Salze überführt.

**5.** Herbizide Mittel, dadurch gekennzeichnet, daß sie Verbindungen der Formel (I) nach einem oder mehreren der Ansprüche 1 bis 3 oder deren Salze und übliche inerte Formulierungshilfsmittel enthalten.

6. Pflanzenwachstumsregulierende Mittel, dadurch gekennzeichnet, daß sie Verbindungen der Formel (I) nach Anspruch 1, 2 oder 3 oder deren Salze und übliche inerte Formulierungshilfsmittel enthalten.

7. Verwendung von nach Anspruch 1, 2 oder 3 definierten Verbindungen der Formel (I) oder deren Salze als Herbizide oder Pflanzenwachstumsregulatoren.

8. Verfahren zur Bekämpfung von unerwünschten Pflanzen, dadurch gekennzeichnet, daß man auf diese oder deren Anbauflächen eine wirksame Menge von Verbindungen der Formel I oder deren Salzen nach Anspruch 1, 2 oder 3 appliziert.

9. Verfahren zur Wachstumsregulierung von Pflanzen, dadurch gekennzeichnet, daß man auf diese oder die Anbauflächen eine wirksame Menge von Verbindungen der Formel I oder deren Salzen nach Anspruch 1, 2 oder 3 appliziert.

**Patentansprüche für folgenden Vertragsstaat : ES**

1. Verfahren zur Herstellung von Verbindungen der Formel (I) oder deren Salzen

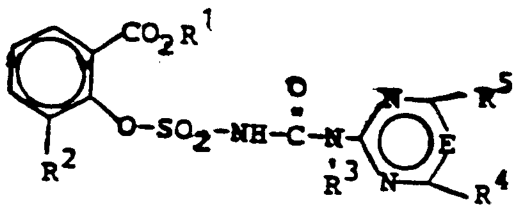

(I)

worin

| | |
|---|---|
| $R^1$ | $(C_1\text{-}C_4)$Alkyl, |
| $R^2$ | Halogen, Methoxy, Ethyl oder Propyl, |
| $R^3$ | Wasserstoff oder Methyl, |
| E | CH oder N und |
| $R^4$, $R^5$ | unabhängig voneinander Halogen, $(C_1\text{-}C_4)$Alkyl, $(C_1\text{-}C_4)$Alkoxy oder $(C_1\text{-}C_4)$Alkylthio, wobei die vorgenannten alkylhaltigen Reste unsubstituiert sind oder ein- oder mehrfach durch Halogen oder ein- oder zweifach durch $(C_1\text{-}C_4)$Alkoxy oder $(C_1\text{-}C_4)$Alkylthio substituiert sind, |

bedeuten, dadurch gekennzeichnet, daß man

a) eine Verbindung der Formel (II)

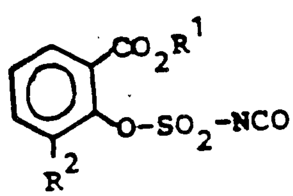

(II)

mit einer Verbindung der Formel (III)

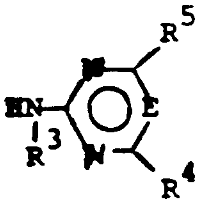

(III)

umsetzt, oder

b) eine Verbindung der Formel (IV)

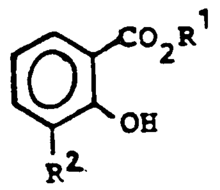

(IV)

mit einem Chlorsulfonylharnstoff der Formel (V)

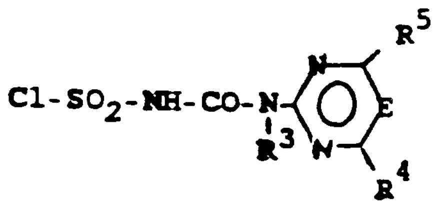

(V)

umsetzt, oder

c) eine Verbindung der Formel (VI)

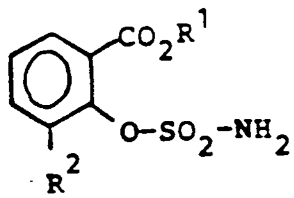

(VI)

mit einem Carbamat der Formel (VII)

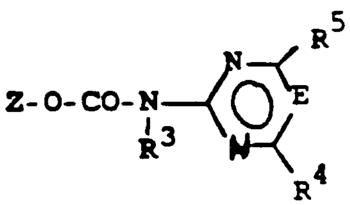

(VII),

worin Z Phenyl oder ($C_1$-$C_6$)Alkyl bedeutet, umsetzt, und die erhaltenen Verbindungen der Formel (I) gegebenenfalls in ihre Salze überführt.

**2.** Verfahren nach Anspruch 1, dadurch gekennzeichnet, daß

| | |
|---|---|
| $R^1$ | Methyl oder Ethyl, |
| $R^2$ | Fluor, Chlor, Methoxy, Ethyl, n-Propyl oder Isopropyl, |
| $R^3$ | Wasserstoff oder Methyl, |
| E | CH und |
| $R^4$ und $R^5$ | unabhängig voneinander Chlor, Brom, ($C_1$-$C_4$)Alkyl oder ($C_1$-$C_4$)Alkoxy, wobei die alkylhaltigen Reste unsubstituiert sind oder ein- oder mehrfach durch Fluor oder Chlor substituiert sind, |

bedeuten.

**3.** Verfahren nach Anspruch 1 oder 2, dadurch gekennzeichnet, daß $R^4$ und $R^5$ unabhängig voneinander Chlor, ($C_1$-$C_2$)Alkyl oder ($C_1$-$C_2$)Alkoxy bedeuten, wobei die vorgenannten alkylhaltigen Reste unsubstituiert sind oder ein- oder mehrfach durch Fluor oder Chlor substituiert sind.

**4.** Verfahren zur Bekämpfung von unerwünschten Pflanzen, dadurch gekennzeichnet, daß man auf diese oder deren Anbauflächen eine wirksame Menge von Verbindungen der in Anspruch 1, 2 oder 3 definierten Formel I oder deren Salzen appliziert.

**5.** Verfahren zur Wachstumsregulierung von Pflanzen, dadurch gekennzeichnet, daß man auf diese oder die Anbauflächen eine wirksame Menge von Verbindungen der in Anspruch 1, 2 oder 3 definierten Formel I oder deren Salzen appliziert.

**6.** Verwendung von Verbindungen der nach Anspruch 1, 2 oder 3 definierten Formel (I) als Herbizide.

**7.** Verwendung von Verbindungen der nach Anspruch 1, 2 oder 3 definierten Formel (I) als Pflanzenwachstumsregulatoren.

**Claims**
**Claims for the following Contracting States : CH, DE, FR, GB, IT, LI**

**1.** A compound of the formula (I) or a salt thereof

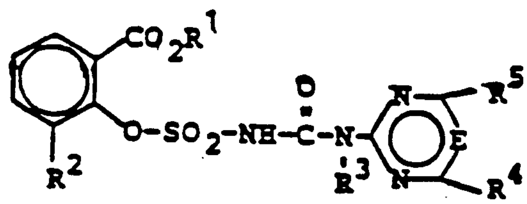

(I)

in which

| | |
|---|---|
| $R^1$ | is $(C_1$-$C_4)$alkyl, |
| $R^2$ | is halogen, methoxy, ethyl or propyl, |
| $R^3$ | is hydrogen or methyl, |
| E | is CH or N and |
| $R^4$ and $R^5$ | independently of one another are halogen, $(C_1$-$C_4)$alkyl, $(C_1$-$C_4)$alkoxy or $(C_1$-$C_4)$-alkylthio, the abovementioned alkyl-containing radicals being unsubstituted or substituted by one or more halogen atoms or one or two $(C_1$-$C_4)$alkoxy or $(C_1$-$C_4)$alkylthio groups. |

**2.** A compound as claimed in claim 1, in which

| | |
|---|---|
| $R^1$ | is methyl or ethyl, |
| $R^2$ | is fluorine, chlorine, methoxy, ethyl, n-propyl or isopropyl, |
| $R^3$ | is hydrogen or methyl, |
| E | is CH and |
| $R^4$ and $R^5$ | independently of one another are chlorine, bromine, $(C_1$-$C_4)$alkyl or $(C_1$-$C_4)$alkoxy, the alkyl-containing radicals being unsubstituted or substituted by one or more fluorine or chlorine atoms. |

**3.** A compound as claimed in claim 1 or 2, in which $R^4$ and $R^5$ independently of one another are chlorine $(C_1$-$C_2)$alkyl or $(C_1$-$C_2)$alkoxy, the abovementioned alkyl-containing radicals being unsubstituted or substituted by one or more fluorine or chlorine atoms.

**4.** A process for the preparation of a compound of the formula (I) as claimed in one or more of claims 1 to 3 or a salt thereof, which comprises

a) reacting a compound of the formula (II)

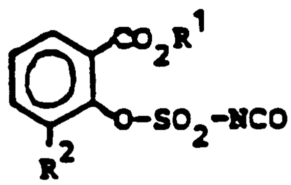

(II)

with a compound of the formula (III)

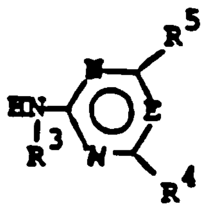

(III)

or

b) reacting a compound of the formula (IV)

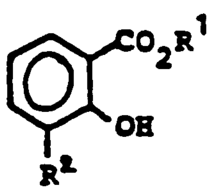

(IV)

with a chlorosulfonylurea of the formula (V)

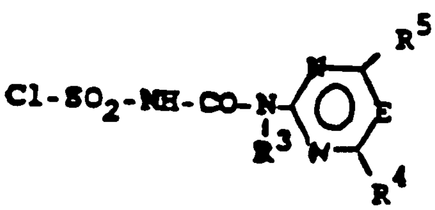

(V)

or

c) reacting a compound of the formula (VI)

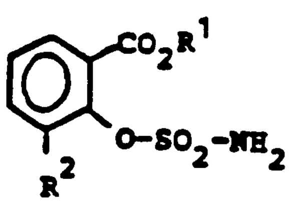

(VI)

with a carbamate of the formula (VII)

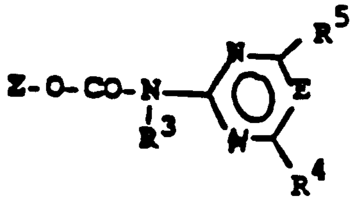

(VII)

in which Z is phenyl or $(C_1-C_6)$alkyl,
and if appropriate converting the resulting compound of the formula (I) into one of its salts.

**5.** A herbicidal agent which contains a compound of the formula (I) as claimed in one or more of claims 1 to 3 or a salt thereof and customary inert formulation auxiliaries.

**6.** A plant growth-regulating agent which contains a compound of the formula (I) as claimed in claim 1, 2 or 3 or a salt thereof and customary inert formulation auxiliaries.

**7.** The use of a compound of the formula (I) defined as claimed in claim 1, 2 or 3 or a salt thereof as a herbicide or plant growth regulator.

**8.** A method for controlling undesirable plants, which comprises applying an effective amount of a compound of the formula I or a salt thereof as claimed in claim 1, 2 or 3 to these plants or their cultivation areas.

**9.** A method of regulating plant growth, which comprises applying an effective amount of a compound of the formula I or a salt thereof as claimed in claim 1, 2 or 3 to these plants or their cultivation areas.

**Claims for the following Contracting State : ES**

**1.** A process for the preparation of a compound of the formula (I) or a salt thereof

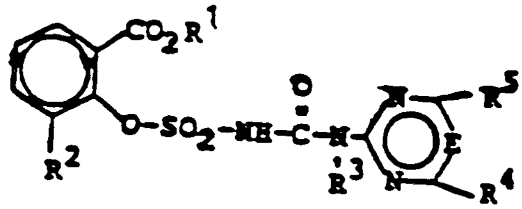

(I)

in which

| | |
|---|---|
| $R^1$ | is $(C_1-C_4)$alkyl, |
| $R^2$ | is halogen, methoxy, ethyl or propyl, |
| $R^3$ | is hydrogen or methyl, |
| E | is CH or N and |
| $R^4$ and $R^5$ | independently of one another are halogen, $(C_1-C_4)$alkyl, $(C_1-C_4)$alkoxy or $(C_1-C_4)$-alkylthio, the abovementioned alkyl-containing radicals being unsubstituted or substituted by one or more halogen atoms or one or two $(C_1-C_4)$alkoxy or $(C_1-C_4)$alkylthio groups, which comprises |

a) reacting a compound of the formula (II)

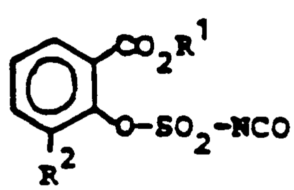

(II)

with a compound of the formula (III)

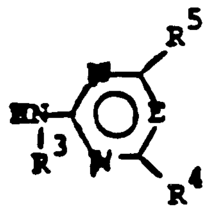

(III)

or

b) reacting a compound of the formula (IV)

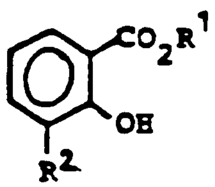

(IV)

with a chlorosulfonylurea of the formula (V)

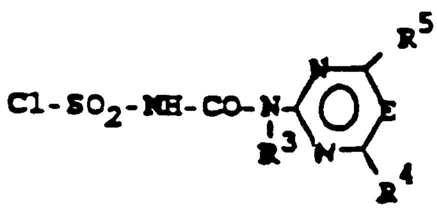

(V)

or

c) reacting a compound of the formula (VI)

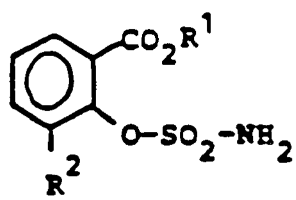

(VI)

with a carbamate of the formula (VII)

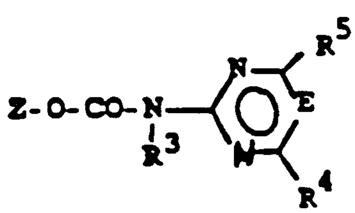

(VII)

in which Z is phenyl or ($C_1$-$C_6$)alkyl,
and if appropriate converting the resulting compound of the formula (I) into one of its salts.

**2.** A compound as claimed in claim 1, in which

$R^1$ is methyl or ethyl,

| | |
|---|---|
| $R^2$ | is fluorine, chlorine, methoxy, ethyl, n-propyl or isopropyl, |
| $R^3$ | is hydrogen or methyl, |
| E | is CH and |
| $R^4$ and $R^5$ | independently of one another are chlorine, bromine, $(C_1-C_4)$alkyl or $(C_1-C_4)$alkoxy, the alkyl-containing radicals being unsubstituted or substituted by one or more fluorine or chlorine atoms. |

3. A compound as claimed in claim 1 or 2, in which $R^4$ and $R^5$ independently of one another are chlorine $(C_1-C_2)$alkyl or $(C_1-C_2)$alkoxy, the abovementioned alkyl-containing radicals being unsubstituted or substituted by one or more fluorine or chlorine atoms.

4. A method for controlling undesirable plants, which comprises applying an effective amount of a compound of the formula I or a salt thereof as claimed in claim 1, 2 or 3 to these plants or their cultivation areas.

5. A method of regulating plant growth, which comprises applying an effective amount of a compound of the formula I or a salt thereof as claimed in claim 1, 2 or 3 to these plants or their cultivation areas.

6. The use of a compound of the formula (I) defined as claimed in claim 1, 2 or 3 as a herbicide.

7. The use of a compound of the formula (I) defined as claimed in claim 1, 2 or 3 as a plant growth regulator.

**Revendications**

**Revendications pour les Etats contractants suivants : CH, DE, FR, GB, IT, LI**

1. Composés de formule (I) ou leurs sels

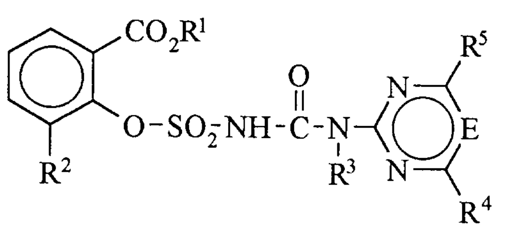

(I)

dans laquelle

| | |
|---|---|
| $R^1$ | représente un reste alkyle en $C_1$-$C_4$, |
| $R^2$ | représente un halogène ou un reste méthoxy, éthyle ou propyle, |
| $R^3$ | représente un hydrogène ou un reste méthyle, |
| E | est CH ou N, |
| $R^4$, $R^5$ | représentent indépendamment l'un de l'autre un halogène ou un reste alkyle en $C_1$-$C_4$, alcoxy en $C_1$-$C_4$ ou alkylthio en $C_1$-$C_4$, les restes alkylés précités étant non substitués ou substitués une ou plusieurs fois par un halogène ou une ou deux fois par un reste alcoxy en $C_1$-$C_4$ ou alkylthio en $C_1$-$C_4$. |

2. Composés selon la revendication 1, caractérisés en ce que

| | |
|---|---|
| $R^1$ | représente un reste méthyle ou éthyle, |
| $R^2$ | représente un atome de fluor ou de chlore ou un reste méthoxy, éthyle, n-propyle ou isopropyle, |
| $R^3$ | représente un hydrogène ou un reste méthyle, |
| E | est CH et |
| $R^4$ et $R^5$ | représentent indépendamment l'un de l'autre un atome de chlore ou de brome ou un reste alkyle en $C_1$-$C_4$ ou alcoxy en $C_1$-$C_4$, les restes alkylés étant non substitués ou substitués une ou plusieurs fois par du fluor ou du chlore. |

3. Composés selon la revendication 1 ou 2, caractérisés en ce que $R^4$ et $R^5$ représentent indépendamment l'un de l'autre un atome de chlore ou un reste alkyle en $C_1$-$C_2$ ou alcoxy en $C_1$-$C_2$, les restes alkylés précités étant non substitués ou substitués une ou plusieurs fois par du fluor ou du chlore.

4. Procédé de préparation des composés de formule générale (I) selon l'une ou plusieurs des revendications 1 à 3 ou de leurs sels, caractérisé en ce que

a) on fait réagir un composé de formule (II)

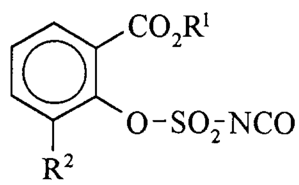

(II)

avec un composé de formule (III)

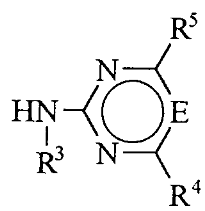

(III)

ou

b) on fait réagir un composé de formule (IV)

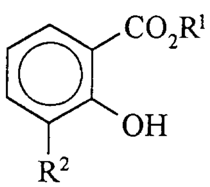

(IV)

avec une chlorosulfonylurée de formule (V)

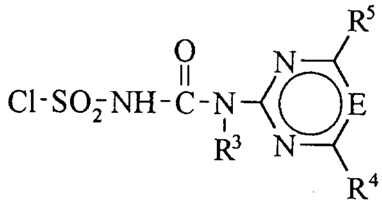

(V)

ou

c) on fait réagir un composé de formule (VI)

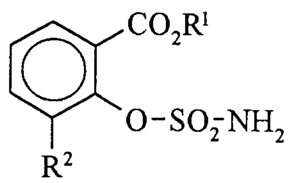

(VI)

avec un carbamate de formule (VII)

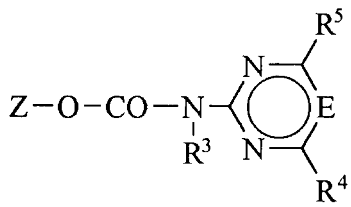

(VII)

dans laquelle Z représente un reste phényle ou alkyle en $C_1$-$C_6$,
et on transforme éventuellement les composés de formule (I) obtenus en leurs sels.

**5.** Compositions herbicides caractérisées en ce qu'elles contiennent des composés de formule (I) selon l'une ou plusieurs des revendications 1 à 3 ou leurs sels et des auxiliaires de formulation inertes classiques.

**6.** Compositions régulatrices de croissance des plantes, caractérisées en ce qu'elles contiennent des composés de formule (I) selon la revendication 1, 2 ou 3 ou leurs sels et des auxiliaires de formulation inertes classiques.

**7.** Utilisation de composés de formule (I) définis selon la revendication 1, 2 ou 3 ou de leurs sels comme herbicides ou régulateurs de croissance des plantes.

**8.** Procédé de lutte contre les plantes indésirables, caractérisé en ce que l'on applique sur ces plantes ou sur leurs surfaces cultivées une quantité active de composés de formule I ou de leurs sels selon la revendication 1, 2 ou 3.

**9.** Procédé de régulation de la croissance de plantes, caractérisé en ce que en ce que l'on applique sur ces plantes ou sur les surfaces cultivées une quantité active de composés de formule I ou de leurs sels selon la revendication 1, 2 ou 3.

**Revendications pour l'Etat contractant suivant : ES**

**1.** Procédé de préparation de composés de formule (I) ou de leurs sels

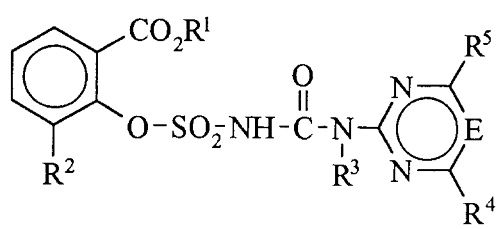

(I)

dans laquelle

$R^1$ représente un reste alkyle en $C_1$-$C_4$,
$R^2$ représente un halogène ou un reste méthoxy, éthyle ou propyle,
$R^3$ représente un hydrogène ou un reste méthyle,
E est CH ou N,
$R^4$, $R^5$ représentent indépendamment l'un de l'autre un halogène ou un reste alkyle en $C_1$-$C_4$, alcoxy en $C_1$-$C_4$ ou alkylthio en $C_1$-$C_4$, les restes alkylés précités étant non substitués ou substitués une ou plusieurs fois par un halogène ou une ou deux fois par un reste alcoxy en $C_1$-$C_4$ ou alkylthio en $C_1$-$C_4$, caractérisé en ce que

a) on fait réagir un composé de formule (II)

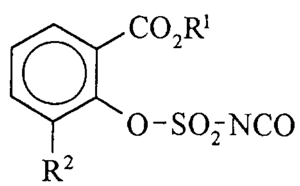

(II)

avec un composé de formule (III)

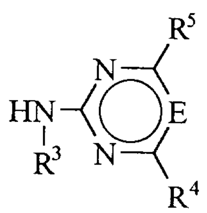

(III)

ou

b) on fait réagir un composé de formule (IV)

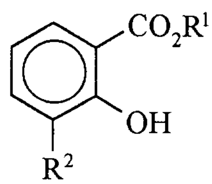

(IV)

avec une chlorosulfonylurée de formule (V)

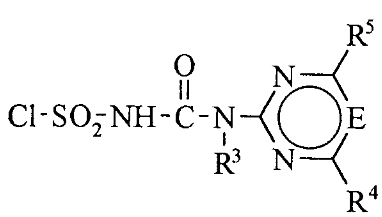

(V)

ou

c) on fait réagir un composé de formule (VI)

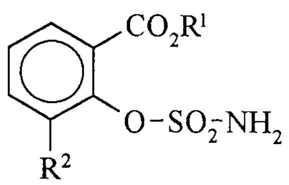

(VI)

avec un carbamate de formule (VII)

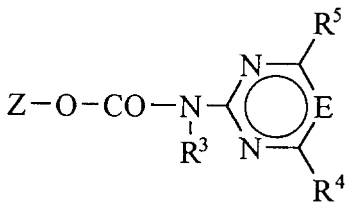

(VII)

dans laquelle Z représente un reste phényle ou alkyle en $C_1$-$C_6$,
et on transforme éventuellement les composés de formule (I) obtenus en leurs sels.

**2.** Procédé selon la revendication 1, caractérisé en ce que

| | |
|---|---|
| $R^1$ | représente un reste méthyle ou éthyle, |
| $R^2$ | représente un atome de fluor ou de chlore ou un reste méthoxy, éthyle, n-propyle ou isopropyle, |
| $R^3$ | représente un hydrogène ou un reste méthyle, |
| E | est CH et |
| $R^4$ et $R^5$ | représentent indépendamment l'un de l'autre un atome de chlore ou de brome ou un reste alkyle en $C_1$-$C_4$ ou alcoxy en $C_1$-$C_4$, les restes alkylés étant non substitués ou substitués une ou plusieurs fois par du fluor ou du chlore. |

**3.** Procédé selon la revendication 1 ou 2, caractérisés en ce que $R^4$ et $R^5$ représentent indépendamment l'un de l'autre un atome de chlore ou un reste alkyle en $C_1$-$C_2$ ou alcoxy en $C_1$-$C_2$, les restes alkylés précités étant non substitués ou substitués une ou plusieurs fois par du fluor ou du chlore.

**4.** Procédé de lutte contre les plantes indésirables, caractérisé en ce que l'on applique sur ces plantes ou sur leurs surfaces cultivées une quantité active de composés ayant la formule I définie dans la revendication 1, 2 ou 3 ou de leurs sels.

**5.** Procédé de régulation de la croissance de plantes, caractérisé en ce que en ce que l'on applique sur ces plantes ou sur les surfaces cultivées une quantité active de composés ayant la formule I définie dans la revendication 1, 2 ou 3 ou de leurs sels.

**6.** Utilisation de composés ayant la formule (I) définie selon la revendication 1, 2 ou 3 comme herbicides.

**7.** Utilisation de composés ayant la formule (I) définie selon la revendication 1, 2 ou 3 comme régulateurs de croissance des plantes.